# EUROPEAN PATENT APPLICATION

(11) **EP 4 578 440 A1**
(43) Date of publication of application: **02.07.2025**
(21) Application number: 23220378.6
(22) Date of filing: 27.12.2023
(51) Int. Cl.: A61K 8/36, A61K 8/41, A61Q 19/04, A61K 8/37

(54) **NON-THERAPEUTIC METHODS FOR SKIN TANNING**

(71) Applicant: IUF Leibnitz-Institut für umweltmedizinische Forschung GmbH, 40225 Düsseldorf (DE)
(72) Inventor: Majora, Marc, 40225 Düsseldorf (DE); Krutmann, Jean, 40225 Düsseldorf (DE)
(74) Representative: dompatent von Kreisler Selting Werner - Partnerschaft von Patent- und Rechtsanwälten mbB

(57) **Abstract**

Non-therapeutic method for imparting a controlled tanning to the skin, comprising the step of applying to the skin a composition comprising a first compound A, which is a methyl group donor, and a second compound B, which is an acetyl-CoA donor.

## Description

The present invention relates to a non-therapeutic method for imparting a controlled tanning to the skin, the non-therapeutic use of compositions comprising a methyl-group donor compound and an acetyl-CoA donor compound for tanning of the skin, and to the use of said compositions in the preparation of skin tanning products.

Suntanning is of social significance in many parts of the world. At least in Western societies, tanning of human skin is viewed as a desirable cosmetic outcome, which is associated with a healthy and attractive physical appearance and high social status. The aesthetic properties of a suntan are primarily due to an increased melanin pigmentation. For cosmetic purposes, one approach to increase skin pigmentation is by exposure to sunlight or ultraviolet (UV) sunlamps, e.g., by using sunbeds or tanning beds. The tanned skin is almost universally regarded as a positive aesthetic attribution, particularly among a high proportion of light-skinned individuals in the Western societies.

The natural color of human skin is among others due to the presence of melanin pigment within the skin, which pigment is usually in the form of discrete granules called melanosomes. The melanosomes are generated in the viable layer of epidermis by pigment cells (melanocytes). This mechanism is called melanogenesis. Specifically, two main types of melanin, eumelanin and pheomelanin, are produced during melanogenesis. Melanogenesis is triggered primarily by UVB radiation, e.g., from sunlight. The UVB radiation triggers an increase in production of melanin.

One method for tanning human skin is exposure to sunlight or artificial UV rays produced by sunlamps. While increasing skin pigmentation by sunlight or artificial UV radiation is effective, it also damages the skin. Especially, premature aging of the skin is observed, which is contradictory to the healthy appearance intended by tanning of the skin. Premature aging of the skin is characterized by sagging, wrinkling, and yellowing of the skin, along with other physical changes such as crackling or lack of elasticity. Despite the disadvantage associated with exposure to sunlight or artificial UV radiation, both sunbed use and excessive sunbathing are still popular among the general population, not least because tanned skin is viewed as a desirable cosmetic outcome associated with a healthy and attractive physical appearance and high social status.

Alternative methods for tanning skin trying to avoid natural or artificial UV irradiation are the use of agents that artificially dye the skin, and the use of agents that increase the natural levels of melanin in the skin.

Presently available agents that act by staining the skin provide an artificial tan without exposure to the sun, but suffer from several disadvantages. It is difficult to match the natural blend of reddish-brown produced by natural tanning, and different shadings on the skin frequently result due to uneven application on the skin. Further, if the tanning agent is applied to the skin by rubbing in a cream or lotion, the palms of the hands often become discolored. Additionally, colored areas are removed non-uniformly during cleaning of the skin. A tan produced by staining lasts only about 5 days, in contrast to the about 4-week duration of a natural suntan.

One example of an artificial tanning agent which has been widely commercialized is dihydroxyacetone. Dihydroxyacetone is not a dye, stain, or paint, but causes a chemical reaction with the amino acids in the dead layer on the skin surface. One of the pathways is a free radical-mediated Maillard reaction. The other pathway is the conventional Maillard reaction, a process that causes the browning that occurs during food manufacturing and storage. Dihydroxyacetone preparations may contain up to 50% alcohol, and such alcoholic preparations tend to dry the skin. Moreover, the color effect is temporary and fades gradually over 3 to 10 days.

To obtain more rapid coloration the art has turned to the use of metallic salts such as copper or ferrous sulfates and indole, a melanin precursor. The metals serve to catalyze the oxidation of the indole and form melanin or melanin-like compounds. Unfortunately, these salts cause the formation of melanin and skin color only in the outermost surface of the skin such that the resulting color is easily removed by ordinary washings. In addition, the color is often gray, contributing to an unpleasant yellow hue of the skin.

At present, there are no methods or compositions available which lead to a satisfactory tanning response in the human skin which is indistinguishable from a natural tan induced by UV irradiation, which do not require exposure of the human skin to UV irradiation.

Thus, there is still the need to provide methods which impart a controlled tanning to the skin having a natural appearance and being indistinguishable from a natural tan induced by UV irradiation.

This object is surprisingly solved by a non-therapeutic method for imparting a controlled tanning to the skin, comprising the step of applying to the skin a composition comprising a first compound A, which is a methyl group donor, and a second compound B, which is an acetyl-CoA donor.

Surprisingly, it has been found that topical application of a combination of a methyl group donor and an acetyl-CoA donor can induce melanogenesis in human skin without the need of artificial UV irradiation. This causes a tanning response which results from an increase in epidermal melanin content and thus provides a tan being indistinguishable from a natural tan induced by UV radiation, but which does not require exposure of human skin to UV radiation.

Advantageously, this offers a possibility to impart a controlled tanning to the skin, which has a natural appearance and lasts as long as a tan induced by (natural) UV irradiation, which is a fully fledged substitute for excessive sunbathing and sunbed use. Moreover, as the skin on the palms of the hands contains fewer melanocytes, the tanning response is less pronounced on the palms. Therefore, if the combination of methyl group donor and acetyl-CoA donor is applied to the skin by rubbing in a cream or lotion, the palms advantageously do not become discolored. In addition, a tanning effect achieved by UV irradiation can be enhanced by the combined use of the methyl group donor and the acetyl-CoA donor, e.g., if the composition is applied to the skin before the skin is exposed to UV radiation from sunlight or from artificial sources. This has the advantage that users no longer have to stay in the sun for as long to achieve the same tanning effect. Excessive sunbathing may thus become less attractive.

In the non-therapeutic method for imparting a controlled tanning to the skin of the present invention, a composition comprising a first compound A, which is a methyl group donor, and a second compound B, which is an acetyl-CoA donor is applied to the skin. The controlled tanning is achieved by the combined application of the first compound A and the second compound B to the skin. Within the meaning of the present invention, "tanning" or "skin tanning" refers to a process whereby the skin color is darkened or tanned. This especially includes "self-tanning", also known as "sunless tanning" or "fake tanning", wherein the effect of a suntan is achieved without exposure to the sun or artificial UV radiation. However, "tanning" or "skin tanning" also includes tanning of the skin by UV radiation (by exposure to UV radiation from sunlight or from artificial sources, like tanning lamps), especially when used in combination with or in addition to the "self-tanning" mentioned before. Within the meaning of the present invention, the term "applied to the skin" means "applied topically" and thus refers to a topical administration of the composition. The method of the present invention is a non-therapeutic method, i.e., an exclusively cosmetic method.

As compound A, any tolerable methyl group donor known from the prior art may be employed. The term "methyl group donor" or "methyl donor" is well known to the person skilled in the art. The term is used in the art to describe compounds which can provide methyl groups in the human body, e.g., in metabolic pathways or DNA synthesis. By way of example, it is pointed to F. Depeint et al. (2006), "Mitochondrial function and toxicity: Role of B vitamins on the one-carbon transfer pathways", Chemico-Biological Interactions, 163, 113-132, and to E.N. Proshkina (2020), "Key Molecular Mechanisms of Aging, Biomarkers, and Potential Interventions", Molecular Biology, 54, 6, 777-811. Exemplary methyl group donors are trimethyl ammonium compounds such as choline and betaine, methionine, folic acid, folate, 5-methyltetrahydrofolate, vitamins B2, B6 and B12, sarcosine, serine, trimethylamine-N-oxide, and S-adenosylmethionine (SAM).

In a preferred embodiment, the first compound A is selected from the group consisting of compounds comprising trimethylamine-groups, sarcosine, trimethylamine-N-oxide, serine, and mixtures of two or more of them. Preferably, the first compound A may be selected from trimethyl ammonium compounds and mixtures of two or more of them. Preferred compounds comprising trimethylamine-groups are choline and betaine. Particularly preferred, the first compound A is selected from choline and betaine. Most preferred is choline as first compound A.

In another preferred embodiment, the first compound A is selected from the group consisting of choline, betaine, sarcosine, trimethylamine-N-oxide, serine, and mixtures of two or more of them, more preferably selected from choline and betaine.

In another preferred embodiment, the first compound A is choline.

As compound B, any tolerable acetyl-CoA donor known from the prior art may be employed. The term "acetyl-CoA donor" is well known to the person skilled in the art. It is used in the art to describe substances or compounds from which acetyl-CoA can be generated in the human body, or substances suppressing compounds which hinder the generation of acetyl-CoA. By way of example, it is pointed to J.M. Frans Trijbels et al. (2004), "Chapter 5 - Biochemical Diagnosis of OXPHOS Disorders" in "Oxidative Phosphorylation in Health and Disease", Eurekah.com and Kluwer Academic / Plenum Publishers. Mixtures of two or more different acetyl-CoA donors can be employed as well. Within the meaning of the present invention, acetyl-CoA donors are substances and compounds from which acetyl-CoA can be generated in the human body, or substances suppressing compounds, such as enzymes, which hinder the generation of acetyl-CoA. Exemplary acetyl-CoA donors are fatty acids, fatty acid derivatives, salts of fatty acids, salts of fatty acid derivatives, triglycerides, triglyceride derivatives, dicarboxylic acid derivatives, and pyruvate dehydrogenase kinase inhibitors.

A compound hindering the generation of acetyl-CoA is for example pyruvate dehydrogenase kinase (PDK). Pyruvate dehydrogenase kinase is a kinase enzyme which acts to inactivate the enzyme pyruvate dehydrogenase by phosphorylating it using ATP. There are four known isozymes of PDK in humans which all catalyze the same reaction. Accordingly, suitable compounds B are also inhibitors of pyruvate dehydrogenase kinases, for example dichloroacetate, AZD7545 ((R)-4-(3-Chloro-4-(3,3,3-trifluoro-2-hydroxy-2-methylpropanamido)phenylsulfonyl)-N,N-dimethylbenzamide), PS10 (2-[(2,4-Dihydroxyphenyl)sulfonyl]isoindoline-4,6-diol), dicoumarol, JX06 (Bis(morpholinothio-carbonyl) disulfide), leelamine or VER-246608 (N-[4-(2-chloro-5-methyl-4-pyrimidinyl)phenyl]-N-[[4-[[(2,2-difluoroacetyl)ami no] methyl] phenyl] methyl]-2,4-dihydroxy-benzam ide).

Fatty acids within the meaning of the present invention are carboxylic acids with 2 to 28 carbon atoms, especially 10 to 25 carbon atoms. Their chain is aliphatic and can be saturated or unsaturated, linear, or branched. Within the meaning of the present invention, both acetic acid and butyric acid are fatty acids. Salts of fatty acids within the meaning of the present invention are cosmetically acceptable salts of fatty acids. Any cosmetically acceptable salt of the above-mentioned fatty acids known from the prior art may be employed. Suitable salts are e.g., sodium salts of fatty acids, potassium salts of fatty acids, ammonium salts of fatty acids, calcium salts of fatty acids or magnesium salts of fatty acids.

Fatty acid derivatives within the meaning of the present invention are modified fatty acids, such as oxylipins, hydroxy fatty acids, diols, alkenones, and wax esters. β-hydroxybutyrate is an example for a fatty acid derivative which may be used in the present invention. Salts of fatty acid derivatives within the meaning of the present invention are cosmetically acceptable salts of fatty acid derivatives. Any cosmetically acceptable salt of the above-mentioned fatty acid derivatives known from the prior art may be employed. Suitable salts are e.g., sodium salts of fatty acid derivatives, potassium salts of fatty acid derivatives, ammonium salts of fatty acid derivatives, calcium salts of fatty acid derivatives or magnesium salts of fatty acid derivatives.

In a preferred embodiment of the invention, the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors. Acetate and butyrate are the salts of acetic acid and butyric acid, respectively. Triheptanoin is an example for a triglyceride which may be used in the present invention. Dimethyl-α-ketoglutarate is an example for a dicarboxylic acid derivative which may be used in the present invention.

In another preferred embodiment, the second compound B is selected from acetate, butyrate, triheptanoin, and dimethyl-α-ketoglutarate, preferably selected from acetate and butyrate.

In another preferred embodiment, the second compound B is acetate.

In a preferred embodiment, the composition consists of the first compound A and the second compound B.

In a particularly preferred embodiment, the composition comprises choline as compound A and an acetate as compound B, or consists thereof.

In another particularly preferred embodiment, the composition comprises choline as compound A and a butyrate as compound B, or consists thereof.

In still another particularly preferred embodiment, the composition comprises choline as compound A and triheptanoin as compound B, or consists thereof.

In still another particularly preferred embodiment, the composition comprises choline as compound A and dimethyl-α-ketoglutarate as compound B, or consists thereof.

Preferably, the molar ratio of the compound A to compound B is in the range of from 10:1 to 1:10, preferably from 8:1 to 1:8, more preferably from 5:1 to 1:5, even more preferably from 2.5:1 to 1:2.5.

In another preferred embodiment, the molar ratio of the compound A to compound B is in the range of from 10:1 to 1:1, preferably from 8:1 to 2:1, more preferably from 5:1 to 2.5:1.

In a preferred embodiment, the compound A is applied to the skin in a concentration in the range of from 1 to 250 mM, preferably in the range of from 5 to 200 mM, more preferably in the range of from 10 to 100 mM, even more preferably in the range of from 25 to 50 mM.

In a preferred embodiment, the compound B is applied to the skin in a concentration in the range of from 1 to 100 mM, preferably in the range of from 5 to 50 mM.

In another preferred embodiment, the compound A is applied to the skin in a concentration in the range of from 1 to 250 mM, preferably in the range of from 5 to 200 mM, more preferably in the range of from 10 to 100 mM, even more preferably in the range of from 25 to 50 mM, and the compound B is applied to the skin in a concentration in the range of from 1 to 100 mM, preferably in the range of from 5 to 50 mM.

In another preferred embodiment, the compound A is applied to the skin in a concentration in the range of from 10 to 100 mM and the compound B is applied to the skin in a concentration of 10 mM.

In the non-therapeutic method of the invention, the composition is applied to the skin. Preferably, the composition is applied to the skin alone or as compound in a cosmetic product. Preferably, the cosmetic product is a skincare product, especially a sunscreen product, a daily skin care product or an anti-aging skin care product.

In general, cosmetics are substances that are intended to come into external contact with the human body (skin, nails, hair). They must serve to cleanse the body, to perfume the body, to change the appearance of the body, to keep the body in good condition or to influence body odor. Within the meaning of the present invention, a "cosmetic product" is a product intended for use as cosmetic within this meaning. In a preferred embodiment, the cosmetic product of the present invention is a product intended for use as skincare product or to alter the appearance. This means that "decorative" cosmetics intended to alter the appearance of the user and "care" cosmetics designed for skincare and personal care are both encompassed by the term "cosmetic product". Examples for "decorative" cosmetics are primers, concealers, foundation, or bronzers. Examples for "care" cosmetics are skincare products like sunscreen products, daily skin care products or anti-aging skin care products. However, it is not excluded that a "decorative" cosmetic may also serve "care" purposes and the other way around.

Preferably, the composition is applied to the skin as compound in a cosmetic product. Since the cosmetic product including the composition is suitable to provide the skin with a sunless tan, the cosmetic product including the composition may be designated as skin tanning product. However, it is to be understood that the cosmetic product can already be a skin tanning product before the addition of the composition, such that any skin tanning effect of the cosmetic product may be supplemented or enhanced by the composition.

Within the meaning of the present invention, a "skin tanning product" is a cosmetic product providing the skin with a sunless tan. An example for a skin tanning product is self-tanner. The terms "skin tanning product" and "self-tanner" encompass both "decorative" cosmetics and "care" cosmetics. Examples for a "care" skin tanning product or a "care" self-tanner are anti-aging self-tanners for daily skin care or skin tanning sunscreen products, i.e., sunscreen products also having self-tanning function.

In a preferred embodiment, the cosmetic product is a skincare product, especially a sunscreen product, a daily skin care product or an anti-aging skin care product. An example for a daily skin care product is moisturizing cream. The skin care products may be in the form of a cream, an emulsion, a lotion, a gel, a spray, a serum, a mousse, a stick, a facial mask, or a body mask, for example.

In another preferred embodiment, the cosmetic product is a decorative cosmetic, preferably a bronzer. Bronzer is a product that adds color to the skin, intended to give the skin a tanned appearance and to enhance the color of the skin. Bronzers are for example used to get a temporary sunless tanning for special occasions, such as festivities. Some athletes, like weightlifters, or bodybuilders use bronzers before sports events or competitions. The bronzer may be in the form of a cream, an emulsion, a lotion, a gel, a spray, a powder, a mousse, a facial mask, or a body mask.

The object of the invention is further solved by a non-therapeutic use of a composition comprising a first compound A, which is a methyl group donor, and a second compound B, which is an acetyl-CoA donor, for tanning of the skin, especially for self-tanning of the skin.

The composition comprising a first compound A, which is a methyl group donor, and a second compound B is used for tanning of the skin, i.e., for imparting a controlled tanning to the skin. The tanning of the skin is achieved by the combined use of the first compound A and the second compound B. The use of the present invention is a non-therapeutic use, i.e., an exclusively cosmetic use of the composition comprising the first compound A and the second compound B.

In a preferred embodiment, the first compound A is selected from the group consisting of compounds comprising trimethylamine-groups, sarcosine, trimethylamine-N-oxide, serine, and mixtures of two or more of them. Preferably, the first compound A may be selected from trimethyl ammonium compounds and mixtures of two or more of them. Preferred compounds comprising trimethylamine-groups are choline and betaine. Particularly preferred, the first compound A is selected from choline and betaine. Most preferred is choline as first compound A.

In another preferred embodiment, the first compound A is selected from the group consisting of choline, betaine, sarcosine, trimethylamine-N-oxide, serine, and mixtures of two or more of them, more preferably selected from choline and betaine.

In another preferred embodiment, the first compound A is choline.

In a preferred embodiment of the invention, the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

In another preferred embodiment, the second compound B is selected from acetate, butyrate, triheptanoin, and dimethyl-α-ketoglutarate, preferably selected from acetate and butyrate.

In another preferred embodiment, the second compound B is acetate.

In a preferred embodiment, the composition consists of the first compound A and the second compound B.

In a particularly preferred embodiment, the composition comprises choline as compound A and an acetate as compound B, or consists thereof.

In another particularly preferred embodiment, the composition comprises choline as compound A and a butyrate as compound B, or consists thereof.

In still another particularly preferred embodiment, the composition comprises choline as compound A and triheptanoin as compound B, or consists thereof.

In still another particularly preferred embodiment, the composition comprises choline as compound A and dimethyl-α-ketoglutarate as compound B, or consists thereof.

Preferably, the molar ratio of the compound A to compound B is in the range of from 10:1 to 1:10, preferably from 8:1 to 1:8, more preferably from 5:1 to 1:5, even more preferably from 2.5:1 to 1:2.5.

In another preferred embodiment, the molar ratio of the compound A to compound B is in the range of from 10:1 to 1:1, preferably from 8:1 to 2:1, more preferably from 5:1 to 2.5:1.

In a preferred embodiment, the compound A is used in a concentration in the range of from 1 to 250 mM, preferably in the range of from 5 to 200 mM, more preferably in the range of from 10 to 100 mM, even more preferably in the range of from 25 to 50 mM.

In a preferred embodiment, the compound B is used in a concentration in the range of from 1 to 100 mM, preferably in the range of from 5 to 50 mM.

In another preferred embodiment, the compound A is used in a concentration in the range of from 1 to 250 mM, preferably in the range of from 5 to 200 mM, more preferably in the range of from 10 to 100 mM, even more preferably in the range of from 25 to 50 mM, and the compound B is used in a concentration in the range of from 1 to 100 mM, preferably in the range of from 5 to 50 mM.

In another preferred embodiment, the compound A is used in a concentration in the range of from 10 to 100 mM and the compound B is used in a concentration 10 mM.

Preferably, in the non-therapeutic use of the present invention, the composition is applied topically, which means applied to the skin. Preferably, the composition is applied topically alone or as compound in a cosmetic product. Preferably, the cosmetic product is a skincare product, especially a sunscreen product, a daily skin care product or an anti-aging skin care product.

Preferably, the composition is applied to the skin as compound in a cosmetic product. Since the cosmetic product including the composition is suitable to provide the skin with a sunless tan, the cosmetic product including the composition may be designated as skin tanning product. However, it is to be understood that the cosmetic product can already be a skin tanning product before the addition of the composition, such that any skin tanning effect of the cosmetic product may be supplemented or enhanced by the composition.

In a preferred embodiment, the cosmetic product is a skincare product, especially a sunscreen product, a daily skin care product or an anti-aging skin care product. The skin care products may be in the form of a cream, a lotion, a gel, a spray, a serum, a mousse, a stick, or a facial mask.

In another preferred embodiment, the cosmetic product is a decorative cosmetic, preferably a bronzer. The bronzer may be in the form of a cream, a lotion, a gel, a spray, a powder, a mousse, or a facial mask.

The object of the invention is further solved by the use of a composition comprising a first compound A, which is a methyl group donor, and a second compound B, which is an acetyl-CoA donor, in the preparation of a skin tanning product, especially in the preparation of a self-tanner.

As already described hereinabove, within the meaning of the present invention, a "skin tanning product" is a cosmetic product providing the skin with a sunless tan. An example for a skin tanning product is self-tanner. The terms "skin tanning product" and "self-tanner" encompass both "decorative" cosmetics and "care" cosmetics. Examples for a "care" skin tanning product or a "care" self-tanner are anti-aging self-tanners for daily skin care or skin tanning sunscreen products, i.e., sunscreen products also having self-tanning function.

If the prepared skin tanning product is applied to the skin, the combination of the first compound A (methyl group donor) and the second compound B (acetyl-CoA donor) induces melanogenesis in the melanocytes of the epidermis and thus leads to a sunless tanning of the skin. Of course, the skin tanning product can also enhance the tanning effect achieved by UV irradiation-induced tanning, e.g., if applied to the skin before the skin is exposed to UV radiation from sunlight or from artificial sources.

In a preferred embodiment, the first compound A is selected from the group consisting of compounds comprising trimethylamine-groups, sarcosine, trimethylamine-N-oxide, serine, and mixtures of two or more of them. Preferably, the first compound A may be selected from trimethyl ammonium compounds and mixtures of two or more of them. Preferred compounds comprising trimethylamine-groups are choline and betaine. Particularly preferred, the first compound A is selected from choline and betaine. Most preferred is choline as first compound A.

In another preferred embodiment, the first compound A is selected from the group consisting of choline, betaine, sarcosine, trimethylamine-N-oxide, serine, and mixtures of two or more of them, more preferably selected from choline and betaine.

In another preferred embodiment, the first compound A is choline.

In a preferred embodiment of the invention, the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

In another preferred embodiment, the second compound B is selected from acetate, butyrate, triheptanoin, and dimethyl-α-ketoglutarate, preferably selected from acetate and butyrate.

In another preferred embodiment, the second compound B is acetate.

In a preferred embodiment, the composition consists of the first compound A and the second compound B.

In a particularly preferred embodiment, the composition comprises choline as compound A and an acetate as compound B, or consists thereof.

In another particularly preferred embodiment, the composition comprises choline as compound A and a butyrate as compound B, or consists thereof.

In still another particularly preferred embodiment, the composition comprises choline as compound A and triheptanoin as compound B, or consists thereof.

In still another particularly preferred embodiment, the composition comprises choline as compound A and dimethyl-α-ketoglutarate as compound B, or consists thereof.

Preferably, the molar ratio of the compound A to compound B is in the range of from 10:1 to 1:10, preferably from 8:1 to 1:8, more preferably from 5:1 to 1:5, even more preferably from 2.5:1 to 1:2.5.

In another preferred embodiment, the molar ratio of the compound A to compound B is in the range of from 10:1 to 1:1, preferably from 8:1 to 2:1, more preferably from 5:1 to 2.5:1.

In a preferred embodiment, the concentration of compound A in the skin tanning product is in the range of from 1 to 250 mM, preferably in the range of from 5 to 200 mM, more preferably in the range of from 10 to 100 mM, even more preferably in the range of from 25 to 50 mM.

In a preferred embodiment, the concentration of compound B in the skin tanning product is in the range of from 1 to 100 mM, preferably in the range of from 5 to 50 mM.

In another preferred embodiment, the concentration of compound A in the skin tanning product is in the range of from 1 to 250 mM, preferably in the range of from 5 to 200 mM, more preferably in the range of from 10 to 100 mM, even more preferably in the range of from 25 to 50 mM, and the concentration of compound B in the skin tanning product is in the range of from 1 to 100 mM, preferably in the range of from 5 to 50 mM.

In another preferred embodiment, the concentration of compound A in the skin tanning product is in the range of from 10 to 100 mM and the concentration of compound B in the skin tanning product is 10 mM.

Preferably, the skin tanning product is selected from the group consisting of self-tanners, skin tanning products for daily skin care, anti-aging self-tanners for daily skin care, and skin tanning sunscreen products. The skin tanning product may serve "decorative" and/or "care" purposes. For example, a skin tanning sunscreen product, which combines the functions of a sunscreen and a self-tanner, on the one hand protects the skin from UV irradiation (sunscreen function) and on the other hand provides the skin with a naturally appearing tan (self-tanner function).

The skin tanning product may be in the form of a cream, an emulsion, a lotion, a gel, a spray, a serum, a mousse, a stick, a facial mask, or a body mask.

The skin tanning product may comprise any suitable additive known by the person skilled in the art, such as solvents, antioxidants, DNA-repair-enzymes, vitamins, UV-filters, pre- or probiotic substances, etc.

The skin tanning product may be prepared by methods known to the person skilled in the art. By way of example, the skin tanning product may be prepared by using an already existing cosmetic product, e.g., a sunscreen, and mixing in the acetyl-CoA donor and the methyl group donor. By way of another example, it is also possible to add the methyl group donor and the acetyl-CoA donor together with the other components when preparing the skin tanning product.

Of course, the prepared skin tanning product may be used in the non-therapeutic method of the present invention and/or used according to the non-therapeutic use of the present invention.

In the following the rationale of the invention is further explained and it is elaborated, how the composition comprising a methyl group donor and an acetyl-CoA donor is linked to skin tanning. These theoretical findings are to be understood as illustrative only and the present invention is not intended to be bound by the following theory.

### Brief description of the Figures

Fig. 1 shows microscope images of skin sections of skin samples obtained from an aged donor, which were cultivated *ex vivo* and received various concentrations of choline (10 - 100 mM) and a fixed concentration of acetate (10 mM) or received control medium according to Example 1.
Fig. 2 shows a Western Blot analysis of proteins isolated from *ex vivo* cultivated skin samples obtained from an aged donor, which received various concentrations of choline (10 - 100 mM) and a fixed concentration of acetate (10 mM) or received control medium according to Example 2.
Fig. 3 shows the relative gene expression level of Tyrp1 in human PIG1 melanocytes, which received various concentrations of choline (10 - 100 mM) and a fixed concentration of acetate (10 mM), or which received control medium, and were either irradiated with UVB or left unirradiated according to Example 3.

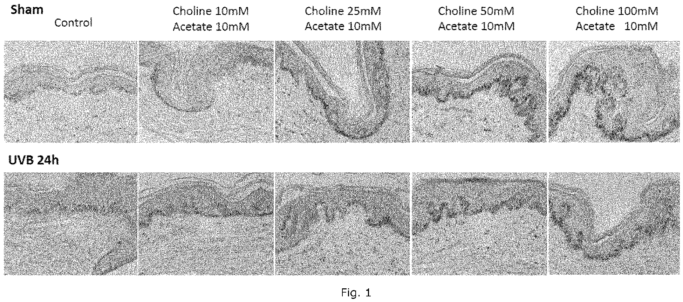

Surprisingly, it has been found that topical application of a methyl group donor in combination with an acetyl-CoA donor can induce melanogenesis in human skin without the need of additional UV irradiation. Topical application of the composition comprising a methyl group donor and an acetyl-CoA donor to human skin from healthy, adult donors caused a significant increase in epidermal melanin content. Accordingly, staining of histological skin sections using a Fontana Masson protocol revealed that a combination of methyl group donor and acetyl-CoA donor increased melanin levels in a dose-dependent manner (Fig. 1). Of note, this tanning response could be elicited without any additional UV irradiation, or, in other words, in unirradiated human skin. A tanning response can also be achieved in UV-preirradiated skin, i.e., application of the combination of methyl group donor and acetyl-CoA donor is capable to further enhance UV-radiation-induced melanin content within the epidermis.

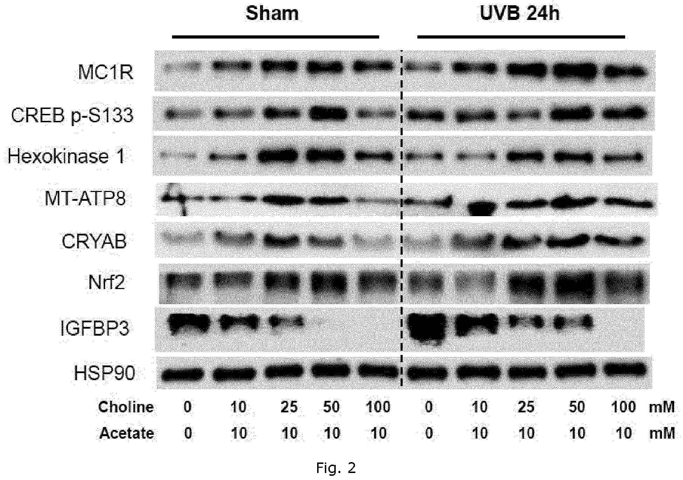

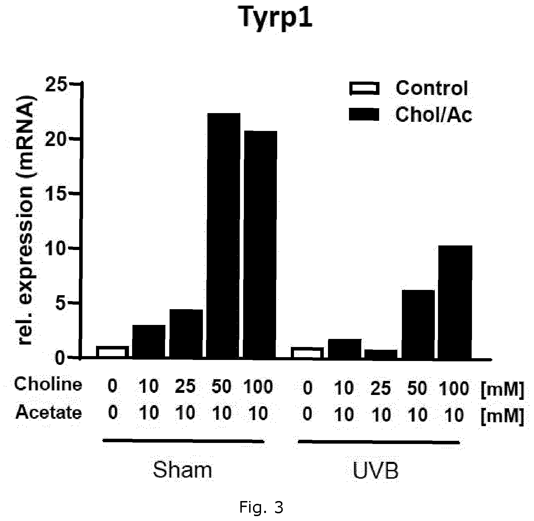

The increase in melanin production was associated with an increased expression of proteins which are known to be critically involved in *de novo* synthesis of melanin in human skin. Accordingly, increased protein levels of the melanocortin 1 receptor (MC1R) and the phosphorylated form of CREB (p-S133) which are both involved in melanin synthesis were found (Fig. 2). Surprisingly, the combined application of methyl group donor and acetyl-CoA donor may also cause melanin synthesis in human melanocytes by modulating the expression of Tyrp1, a gene responsible for the synthesis of the UV-protective pigment eumelanin in melanocytes (Fig. 3). Taken together these results show that topical application of the combination of methyl group donor and acetyl-CoA donor can induce a tanning response in human skin. This tanning response is due to an increase in epidermal melanin content, and thus is indistinguishable from a natural tan induced by UV rays and lasts as long as a natural tan.

It is to be understood that preferred embodiments or preferred features mentioned for one embodiment are also to be applied in the same way for all other embodiments mentioned in the application. Each preferred embodiment and each preferred feature can be combined with each other without further limitation.

In the following examples, the present invention is disclosed but without limiting the scope of protection to this specific examples.

### EXAMPLES

### Description of the materials and methods used in Examples 1 to 3

### Ex vivo skin culture and treatment

A human skin sample derived from a female 57-year-old donor undergoing skin surgery was received from a local hospital. After being placed under a laminar flow hood, the skin sample was shortly disinfected in 70% EtOH, washed three times and kept in phosphate buffered saline (PBS) containing 100 U/ml penicillin, 100 µg/ml streptomycin and 0.25 µg/ml amphotericin B. The skin sample was cut into smaller pieces of equal size and the subcutaneous fat tissue was removed using scalpels and scissors.

For skin culture, a sterile surgical SMI Spon gelatin sponge (SMI AG, Belgium) was cut into small equal pieces using a scalpel. The sponge pieces were put into six-well-plates and allowed to soak with culture medium [DMEM / Ham's F12-Medium (1:1), 2% FCS, 1x Glutamax, 1x antibiotics/antimycotics] containing varying concentrations of choline chloride (10, 25, 50 or 100 mM) and a constant concentration of sodium acetate (10 mM) (both chemicals were purchased from Sigma-Aldrich). Control medium used here did not contain any added choline chloride or sodium acetate. Skin pieces were placed on the surface of the soaked sponges and cultivated for 24 h.

For UVB exposure, culture medium was removed and skin pieces were irradiated with UVB (dose 50 mJ/cm²). After irradiation, fresh corresponding medium was added and the skin pieces were cultivated for additional 24 h.

### Histological processing and Fontana Masson staining

Following the treatment, skin pieces were fixed in 4% paraformaldehyde at 4 °C overnight. After being dehydrated, skin samples were embedded in paraffin and cut into thin sections using a microtome. Fontana Masson staining was done using a commercially available kit (ScyTek Laboratories, USA) according to the protocol of the manufacturer. Briefly, sections were deparaffinized in citrate buffer and rehydrated before being incubated in ammoniacal silver solution at 60 °C for 45 min. After three times of washing in distilled H₂O, slides were incubated in gold chloride solution (0.2%) for 30 sec. Slides were again washed three times with distilled water before being incubated in sodium thiosulfate solution (5%) for 2 min. After being rinsed under running tap water for 2 min, slides were washed twice with distilled water. Sections were counterstained using Nuclear Fast Red solution for 5 min, rinsed for 2 min under running tap water and washed twice with distilled water. Using three changes of absolute ethanol, sections were dehydrated before being mounted in synthetic resin and being analyzed with a light microscope.

### Protein extraction and Western blot analysis

After treatment, skin pieces were transferred into 2 ml Eppendorf tubes filled with ice cold RIPA lysis and protein extraction buffer supplied with proteinase and phosphatase inhibitors. A small stainless-steel bead (diameter: 3 mm) was added to each tube and the samples were shredded using a TissueLyser II (Qiagen). Samples were kept on ice for 30 minutes before being spun down at 14000 rpm for 15 minutes at 4 °C. Supernatants with solubilized proteins were collected, snap frozen in liquid nitrogen and stored at -80 °C. For Western Blot detection, protein aliquots were separated by SDS-polyacrylamide gel electrophoresis before being transferred on PVDF membranes by using the Trans-Blot Turbo Transfer system (Bio-Rad). Membrane slices were incubated with the indicated antibodies overnight on a shaker at 4 °C. The following antibodies were used: CREB p-S133 (Cell Signaling, #9198), CRYAB (Novus, #NBP1-22410), Hexokinase 1 (Cell Signaling, #2024), HSP90 (Cell Signaling, #4877), IGFBP3 (Cell Signaling, #64143), MC1R (Invitrogen, #PA5-21911), MT-ATP8 (Genetex, #GTX55993), Nrf2 (Novus, #NBP1-32822). After washing, membrane slices were incubated with appropriate secondary antibodies coupled to horseradish peroxidase for 2 hours at room temperature. After washing, membrane slices were incubated with ECL western blotting substrate and protein bands were detected with an Odyssey XF Imaging system (LI-COR).

### Cell Culture and treatment

Human PIG1 melanocytes were grown on culture dishes in melanocyte growth medium containing 5% FCS. 48 hours before UVB irradiation, medium was removed, cells were washed with phosphate buffered saline (PBS) and received growth medium (Ham's F-10 containing 10 ng/ml Tetradecanoyl-Phorbol 13-Acetat (TPA), 100 µM IBMX, 1% Ultroser G and 1x antibiotics/antimycotics). 24 hours later, medium was removed and cells received medium containing varying concentrations of choline chloride (10, 25, 50 or 100 mM) and a constant concentration of sodium acetate (10 mM). Control medium used here did not contain any extra choline chloride or sodium acetate. 24 hours later, medium was removed, cells were washed with PBS and were irradiated with UVB (dose: 60 mJ/cm²) in fresh PBS. Unirradiated control (sham) cells were treated essentially equal but were not exposed to UVB. After irradiation, PBS was removed, cells were treated with the corresponding medium and were cultivated for additional 24 hours.

### RNA isolation & Real-Time PCR

24 hours after irradiation, medium of PIG1 melanocytes was removed, cells were washed with PBS and RNA was extracted using a Direct-zol RNA kit (Zymo Research) according to the protocol of the manufacturer and stored at -80 °C. 200 ng of purified RNA were used for cDNA synthesis using SuperScript reverse transcriptase (Thermo Fisher). Expression level of the gene Tyrp1 was analyzed using a CFX Connect Real-Time PCR detection system and SYBR Green Supermix (both from Bio-Rad). Expression level of Tyrp1 was normalized in comparison to the housekeeper gene TFPI2.

### Example 1

Skin samples obtained from an aged donor were cultivated *ex vivo* and received various concentrations of choline (10 - 100 mM) and a fixed concentration of acetate (10 mM) or received control medium.] After 48 h of cultivation, skin samples were irradiated with UVB or left unirradiated. 24 h later skin samples were fixed in paraformaldehyde and embedded in paraffin before being cut into sections. The skin sections were subjected to a Fontana Masson staining protocol using a commercially available kit (detailed description of the experimental procedure: see above).

The images shown in Fig. 1 indicate a dose-dependent increase of melanin content by treatment with choline/acetate with and without UVB irradiation. The higher the dose of choline/acetate, the more cells within the respective skin sections show dark brown or black color. The cells with the dark brown or black color located in the bottom layer (the stratum basale) of the epidermis are the melanocytes, and the more intense their color, the more melanin was produced by melanogenesis. Surprisingly, the melanocytes of the skin sample treated with 10 mM choline and 10 mM acetate, but not irradiated with UVB, have a similar amount of melanin like the melanocytes of the skin sample not treated with choline and acetate, but irradiated with UVB.

### Example 2

Skin samples obtained from an aged donor were cultivated *ex vivo* and received various concentrations of choline (10 - 100 mM) and a fixed concentration of acetate (10 mM) or received control medium. After 48 h of cultivation, skin samples were irradiated with UVB or left unirradiated and proteins were extracted 24 h after irradiation. Proteins were separated by SDS-polyacrylamide gel electrophoresis and transferred to a PVDF membrane which then was probed with antibodies raised against the indicated markers (detailed description of the experimental procedure: see above).

As shown in Fig. 2, MC1R and CREB p-S133, which are involved in the synthesis of melanin, are increased by choline/acetate, in particular by a choline concentration of 50 mM (in combination with 10 mM acetate) and without UVB irradiation. Hexokinase 1, MT-ATP8, CRYAB and Nrf2 are in particular upregulated at choline concentrations of 25 and 50 mM (in combination with 10 mM acetate, respectively) in both UVB-irradiated and non-irradiated skin samples. The senescence-associated marker IGFBP3 is increased after UVB irradiation and shows a dose-dependent downregulation with and without UVB. HSP90 was used as reference.

### Example 3

Human PIG1 melanocytes received varying concentrations of choline (10 - 100 mM) and a constant concentration of acetate (10 mM). Control cells just received medium without any added choline and acetate. 24 h later, cells were either irradiated with UVB or left unirradiated (Sham). Expression of the melanocyte-associated gene Tyrp1 was analyzed 24 h after UVB irradiation in relation to the housekeeping gene TFPI2 (detailed description of the experimental procedure: see above).

As shown in Fig. 3, choline/acetate leads to a dose-dependent increase of Tyrp1 RNA levels in human PIG1 melanocytes with and without UVB irradiation. This effect is especially pronounced in non-irradiated cells. Tyrp1 is responsible for the synthesis of the UV-protective pigment eumelanin in melanocytes. Thus, these data indicate that choline/acetate may directly affect melanin synthesis in human melanocytes by modulating the expression of Tyrp1.

## Claims

1. Non-therapeutic method for imparting a controlled tanning to the skin, comprising the step of applying to the skin a composition comprising a first compound A, which is a methyl group donor, and a second compound B, which is an acetyl-CoA donor.

2. Non-therapeutic method according to claim 1, wherein
(i) the first compound A is selected from the group consisting of compounds comprising trimethylamine-groups, sarcosine, trimethylamine-N-oxide, serine, and mixtures of two or more of them, preferably selected from trimethyl ammonium compounds and mixtures of two or more of them, especially selected from choline and betaine; and/or
(ii) the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

3. Non-therapeutic method according to claim 1 or 2, wherein the molar ratio of compound A to compound B is from 10:1 to 1:10, preferably from 8:1 to 1:8, more preferably from 5:1 to 1:5, even more preferably from 2.5:1 to 1:2.5.

4. Non-therapeutic method according to any of claims 1 to 3, wherein
(i) the compound A is applied to the skin in a concentration in the range of from 1 to 250 mM, preferably in the range of from 5 to 200 mM, more preferably in the range of from 10 to 100 mM, even more preferably in the range of from 25 to 50 mM; and/or
(ii) the compound B is applied to the skin in a concentration in the range of from 1 to 100 mM, preferably in the range of from 5 to 50 mM.

5. Non-therapeutic method according to any one of claims 1 to 4, wherein the composition is applied to the skin alone or as compound in a cosmetic product, preferably a skincare product, especially a sunscreen product, a daily skin care product or an anti-aging skin care product.

6. Non-therapeutic use of a composition comprising a first compound A, which is a methyl group donor, and a second compound B, which is an acetyl-CoA donor, for tanning of the skin, especially for self-tanning of the skin.

7. Non-therapeutic use according to claim 6, wherein
(i) the first compound A is selected from the group consisting of compounds comprising trimethylamine-groups, sarcosine, trimethylamine-N-oxide, serine, and mixtures of two or more of them, preferably selected from trimethyl ammonium compounds and mixtures of two or more of them, especially selected from choline and betaine; and/or
(ii) the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

8. Non-therapeutic use according to claim 6 or 7, wherein the molar ratio of compound A to compound B is from 10:1 to 1:10, preferably from 8:1 to 1:8, more preferably from 5:1 to 1:5, even more preferably from 2.5:1 to 1:2.5.

9. Non-therapeutic use according to any of claims 6 to 8, wherein
(i) the compound A is used in a concentration in the range of from 1 to 250 mM, preferably in the range of from 5 to 200 mM, more preferably in the range of from 10 to 100 mM, even more preferably in the range of from 25 to 50 mM; and/or
(ii) the compound B is used in a concentration in the range of from 1 to 100 mM, preferably in the range of from 5 to 50 mM.

10. Non-therapeutic use according to any one of claims 6 to 9, wherein the composition is applied topically, preferably alone or as compound in a cosmetic product, especially a skincare product, especially a sunscreen product, a daily skin care product or an anti-aging skin care product.

11. Use of a composition comprising a first compound A, which is a methyl group donor, and a second compound B, which is an acetyl-CoA donor, in the preparation of a skin tanning product, especially in the preparation of a self-tanner.

12. Use according to claim 11, wherein
(i) the first compound A is selected from the group consisting of compounds comprising trimethylamine-groups, sarcosine, trimethylamine-N-oxide, serine, and mixtures of two or more of them, preferably selected from trimethyl ammonium compounds and mixtures of two or more of them, especially selected from choline and betaine; and/or
(ii) the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

13. Use according to claim 11 or 12, wherein the molar ratio of compound A to compound B is from 10:1 to 1:10, preferably from 8:1 to 1:8, more preferably from 5:1 to 1:5, even more preferably from 2.5:1 to 1:2.5.

14. Use according to any of claims 11 to 13, wherein
(i) the concentration of compound A in the skin tanning product is in the range of from 1 to 250 mM, preferably in the range of from 5 to 200 mM, more preferably in the range of from 10 to 100 mM, even more preferably in the range of from 25 to 50 mM; and/or
(ii) the concentration of compound B in the skin tanning product is in the range of from 1 to 100 mM, preferably in the range of from 5 to 50 mM.

15. Use according to any one of claims 11 to 14, wherein the skin tanning product is selected from the group consisting of self-tanners, skin tanning products for daily skin care, anti-aging self-tanners for daily skin care, and skin tanning sunscreen products.
